# DEMANDE DE BREVET EUROPEEN

(11) **EP 0 873 697 A1**
(43) Date de publication de la demande: **28.10.1998**
(21) Numéro de dépôt: 98400961.3
(22) Date de dépôt: 20.04.1998
(51) Int. Cl.: A45C 11/00, G02C 13/00, A61L 2/18

(54) **Etui plat pour la désinfection de lentilles de contact**

(30) Priorité: 22.04.1997 FR 9704937
(71) Demandeur: ESSILOR INTERNATIONAL (COMPAGNIE GENERALE D'OPTIQUE), F-94227 Charenton cédex (FR)
(72) Inventeur: Bourset, Claude, 94000 Creteil (FR)
(74) Mandataire: Casalonga, Axel

(57) **Abrégé**

L'étui plat comprend un corps d'étui (1) comportant au moins une paroi (2) définissant une chambre de désinfection (10) ouverte à une extrémité et destinée à recevoir une lentille (50) à désinfecter, au moins un bouchon amovible (20) destiné à être fixé sur l'extrémité ouverte de la chambre, au moins un élément catalytique solidaire du bouchon (20) apte à plonger dans la solution de désinfection (10) et comprenant un support de catalyseur et un catalyseur (34) et au moins un élément de protection (30) solidaire du bouchon (20) ayant une paroi de fond (32) pourvue de passages (33) pour la solution de désinfection, faisant barrière entre la lentille (50) et l'élément catalytique tout en assurant un bon maintien de la lentille.

Application à la désinfection des lentilles de contact.

## Description

La présente invention concerne d'une manière générale un étui plat pour la désinfection de lentilles de contact, et plus particulièrement un tel étui plat dans lequel les lentilles de contact sont désinfectées au moyen d'une solution de désinfection, par exemple une solution oxydante à base de péroxyde d'hydrogène et dans lequel la désinfection des lentilles de contact et la neutralisation de la solution de désinfection s'effectuent simultanément.

Il est connu, pour la désinfection des lentilles de contact, d'immerger les lentilles de contact dans une solution de désinfection oxydante à base de péroxyde d'hydrogène.

Il va de soi qu'il est extrêmement dangereux d'insérer dans l'oeil une lentille de contact venant juste d'être retirée d'une solution désinfectante de péroxyde d'hydrogène, car une telle solution désinfectante est fortement irritante pour l'oeil. Par conséquent, la solution désinfectante doit être neutralisée avant la réutilisation des lentilles de contact. Cette étape de neutralisation s'effectue de façon commode en mettant en contact un catalyseur de décomposition du péroxyde d'hydrogène avec la solution de péroxyde d'hydrogène.

On a également proposé des procédés et des ensembles de désinfection de lentilles de contact dans lesquels on mettait simultanément en contact avec une solution de désinfection, telle qu'une solution de péroxyde d'hydrogène, les lentilles de contact et le catalyseur de neutralisation de telle sorte que la réaction de neutralisation de la solution de désinfection commençait en même temps que la décontamination des lentilles de contact.

Le document EP-A-354 876 décrit un procédé et un ensemble de désinfection de lentilles de contact dans lesquels la désinfection des lentilles de contact et la neutralisation de la solution de désinfection s'effectuent simultanément. L'ensemble de désinfection du document EP-A-354 876 comprend un récipient cylindrique et un couvercle détachable monté sur le récipient. Un support de lentilles de contact et de bloc de catalyseur solidaire du bouchon pend à partir de ce couvercle dans le récipient. Avec cet ensemble de désinfection, la solution de désinfection, par exemple une solution de péroxyde d'hydrogène, est introduite dans le récipient, puis le bloc de catalyseur et les lentilles de contact sont introduits simultanément dans la solution lorsque l'on fixe le couvercle sur l'extrémité du récipient. Dans une réalisation du document EP-A-354 876, le bloc de catalyseur est contenu dans un logement réalisé dans le support de lentilles de contact sur un côté des paniers destinés à maintenir les lentilles de contact. Une telle réalisation conduit à un support de lentilles de contact et de bloc de catalyseur complexe.

Dans une autre réalisation du document EP-A-354 876, le bloc de catalyseur est simplement monté à l'extrémité inférieure du support de lentilles de contact, en dessous des paniers de maintien des lentilles de contact. Avec une telle construction du support de lentilles de contact et de bloc de catalyseur, le bloc de catalyseur se trouve exposé à un contact accidentel lorsque le couvercle et le support de lentilles de contact et de bloc de catalyseur sont retirés du récipient.

Dans le document EP-A-560 728, on décrit un ensemble de désinfection de lentilles de contact comprenant un corps d'étui cylindrique et un couvercle auquel se trouve fixé un support de lentilles de contact. Un catalyseur est disposé dans la partie supérieure du corps d'étui sur la surface interne de celui-ci. Un agitateur, par exemple un agitateur magnétique à ailette, est disposé au fond du corps d'étui. Lors de l'utilisation, on remplit le corps d'étui avec une solution oxydante de péroxyde d'hydrogène jusqu'à un niveau situé au-dessus du catalyseur, puis on introduit le support de lentilles de contact contenant les lentilles de contact à désinfecter en fixant le couvercle sur l'extrémité ouverte du corps d'étui. Enfin, on agite la solution de péroxyde en mettant en rotation l'agitateur.

Dans cette réalisation du document EP-A-560 728, la solution oxydante de désinfection est initialement introduite jusqu'à remplir le corps d'étui au-dessus du catalyseur, de sorte que la réaction de neutralisation de la solution oxydante débute avant que le support de lentilles de contact, et par conséquent les lentilles de contact à désinfecter, ne soit introduit dans le corps d'étui. De ce fait, il existe un risque que la solution oxydante soit neutralisée avant l'introduction du support de lentilles de contact et des lentilles de contact à désinfecter, à un point tel que la solution oxydante de désinfection n'ait plus l'efficacité suffisante pour désinfecter de façon appropriée les lentilles de contact.

En outre, dans les réalisations des documents EP-A-354 876 et EP-A-560 728, les ensembles sont relativement encombrants et le catalyseur se trouve relativement éloigné des lentilles à désinfecter.

Il est souhaitable, pour assurer une neutralisation la plus complète possible au voisinage des lentilles, ainsi qu'une agitation mécanique de celles-ci sous l'effet des bulles d'oxygène libérées lors de la neutralisation, de disposer le catalyseur le plus près possible des lentilles.

Le document US-A-5 431 879 décrit un étui plat pour la désinfection de lentilles de contact comprenant un corps d'étui comportant deux logements de réception et deux bouchons de fermeture des logements de réception. Les lentilles à désinfecter sont maintenues dans le logement par deux porte-lentilles perforés fixés aux parois latérales des logements. Les lentilles sont stérilisées et désinfectées au moyen de solutions d'agents anti-microbiens. Dans une réalisation, des éléments de "neutralisation" de la solution anti-microbienne sont disposés sur le fond des logements de réception afin d'extraire l'agent anti-microbien une fois la stérilisation ou la désinfection achevée.

La présente invention a donc pour objet de fournir un étui plat pour la désinfection de lentilles de contact dans lequel la désinfection des lentilles de contact s'effectue simultanément avec la neutralisation de la solution de désinfection, qui soit de faible encombrement et de construction simple, qui protège le catalyseur d'un contact accidentel, en particulier avec les doigts d'un utilisateur ou tout autre objet, et qui assure que la neutralisation de la solution de désinfection ne débute que lorsque les lentilles de contact sont immergées dans la solution de désinfection.

La présente invention a également pour objet de fournir un étui plat pour la désinfection de lentilles de contact comportant des moyens assurant un maintien approprié des lentilles et également une protection vis-à-vis d'un contact éventuel avec le catalyseur.

La présente invention a encore pour objet de fournir un étui plat tel que défini ci-dessus, dans lequel le catalyseur se trouve disposé le plus près possible des lentilles pour assurer une neutralisation la plus complète possible de la solution de désinfection au voisinage des lentilles et une agitation mécanique de celles-ci.

Selon l'invention, on réalise un étui plat pour la désinfection de lentilles de contact ayant une face convexe et une face concave au moyen d'une solution de désinfection, par exemple une solution oxydante à base de péroxyde d'hydrogène, qui comprend :
- un corps d'étui comportant au moins une paroi définissant une chambre de désinfection, de préférence de forme générale hémisphérique correspondant à la face convexe de la lentille, ouverte à une extrémité, destinée à contenir la solution de désinfection et à recevoir une lentille à désinfecter;
- au moins un bouchon amovible destiné à être fixé sur l'extrémité ouverte de la chambre de désinfection;
- au moins un élément catalytique de neutralisation de la solution de désinfection solidaire du bouchon, apte à plonger dans la solution de désinfection lorsque le bouchon est fixé au corps d'étui, et comprenant un support de catalyseur et un catalyseur; et
- au moins un élément de protection solidaire du bouchon, ayant une paroi de fond pourvue de passages pour la solution de désinfection, faisant barrière entre la lentille et l'élément catalytique, tout en assurant un bon maintien de la lentille.

De préférence, l'étui plat comprend un corps d'étui comportant deux parois distinctes définissant deux chambres de désinfection séparées, deux bouchons, deux éléments catalytiques et deux éléments de protection.

Les éléments de protection peuvent être fixés à l'intérieur des bouchons, par exemple par encliquetage ou être moulés d'une seule pièce avec les bouchons. Les éléments de protection peuvent également chacun être fixés à un joint annulaire d'étanchéité, lui-même fixé, par exemple par collage, à l'intérieur du bouchon.

Les éléments de protection solidaires des bouchons peuvent avoir toute forme appropriée pour autant qu'ils plongent dans la solution de désinfection lorsque les bouchons sont fixés au corps d'étui et qu'ils permettent la circulation de la solution de désinfection et en particulier permettent à la solution de désinfection d'être en contact avec tout ou la majeure partie du catalyseur.

Dans une réalisation recommandée de l'invention, les éléments de protection, assurant le maintien des lentilles dans les chambres de désinfection, ont une paroi de fond de forme générale hémisphérique, de préférence correspondant à la face concave des lentilles.

Dans une réalisation de l'invention, le support de catalyseur de l'élément catalytique est constitué par les surfaces non exposées ou internes de la paroi de fond de l'élément de protection, c'est-à-dire toutes les surfaces autres que la surface de l'élément de protection faisant face à la lentille à désinfecter, et sur lesquelles est déposée une couche de catalyseur, ce qui protège le catalyseur de tout contact dommageable.

Dans une autre réalisation de l'invention, le support de catalyseur de l'élément catalytique est constitué par un élément rapporté fixé à l'intérieur de l'élément de protection et comportant des éléments saillants, orientés vers la paroi de fond de l'élément de protection, et sur lesquels est déposé le catalyseur. De préférence, les éléments saillants ont une hauteur qui croît depuis la périphérie de l'élément rapporté vers son centre, en particulier lorsque la paroi de fond de l'élément de protection est de forme hémisphérique. Cette réalisation assure une protection efficace du catalyseur contre tout contact dommageable.

A titre d'exemple, la paroi de fond de l'élément de protection, de préférence de forme hémisphérique, permettant le passage de la solution de désinfection, peut être formée d'un élément perforé, d'un treillis, d'un élément à croisillons ou toute autre structure permettant le passage et la circulation de la solution de désinfection, tout en assurant un maintien de la lentille dans la chambre de désinfection.

Les catalyseurs de neutralisation des solutions oxydantes à base de péroxyde d'hydrogène sont bien connus dans la technique.

On peut utiliser dans la présente invention tous catalyseurs de neutralisation des solutions de désinfection, par exemple à base de péroxyde d'hydrogène. Parmi les catalyseurs recommandés, utiles dans la présente invention, on peut citer les métaux du groupe du platine, tels que le platine et le palladium, en particulier le noir de platine, et les enzymes ou catalases immobilisées sur un support, par exemple comme décrit dans les documents EP-A-209 071 et WO-86 07 264.

Le catalyseur est en général déposé directement sur les surfaces choisies du support de catalyseur de l'élément catalytique.

Lorsque le catalyseur de l'élément catalytique est constitué par un métal du groupe du platine, il peut être déposé par dépôt chimique en phase vapeur ou par pulvérisation cathodique.

Les solutions oxydantes de désinfection à base de péroxyde d'hydrogène sont bien connues dans la technique et sont décrites entre autres dans la demande de brevet français n° 76 24 837.

Le corps d'étui, les bouchons, les éléments de protection et les supports de catalyseurs lorsqu'ils sont distincts des éléments de protection sont réalisés, de préférence en matière plastique, par exemple en polyoxyphénylène, polypropylène ou autres.

Lorsque l'élément de protection a une paroi de fond de forme hémisphérique recommandée avec sa face convexe disposée en regard de la paroi de fond de la chambre de désinfection, non seulement il assure un maintien de la lentille et une protection efficace du catalyseur, mais également il permet de disposer le catalyseur, que son support soit constitué par les surfaces internes de l'élément de protection ou par un élément rapporté, au plus près de la face concave de la lentille. Ainsi, il est possible d'effectuer une neutralisation plus complète au voisinage immédiat de la lentille. En outre, comme la réaction de neutralisation de la solution de désinfection produit un dégagement d'oxygène, les bulles d'oxygène qui sont produites à proximité immédiate de la lentille, provoquent une agitation de la solution de désinfection et de la lentille assurant une meilleure désinfection de la lentille et simultanément une meilleure neutralisation de la solution de désinfection.

La suite de la description se réfère aux figures annexées qui représentent :
figure 1 - une vue en coupe d'une réalisation d'un étui plat, selon l'invention;
figure 2 - une vue en coupe d'un étui plat analogue à celui de la figure 1, dans lequel l'étanchéité et le dégazage sont assurés par une lèvre souple à la place d'un joint annulaire classique;
figure 3 - une vue en coupe d'une autre réalisation d'un étui plat, selon l'invention;
figure 4 - une vue en coupe d'un élément de protection de l'étui plat de la figure 1;
figure 5 - une vue de dessous de l'élément de protection de la figure 4;
figure 6 - une vue en coupe d'une autre réalisation d'un élément de protection, selon l'invention;
figure 7 - une vue de dessous de l'élément de protection de la figure 6;
figure 8 - une vue en coupe d'encore une autre réalisation d'un élément de protection, selon l'invention, à l'intérieur duquel est fixé un élément rapporté servant de support de catalyseur;
figure 9 - une vue de dessous d'un élément rapporté servant de support de catalyseur de la figure 8;
figure 10 - une vue de dessous d'une variante de l'élément rapporté servant de support de catalyseur de la figure 8;
figure 11 - une vue en coupe d'un moyen de dégazage, avant son montage; et
figure 12 - une vue en coupe du moyen de dégazage de la figure 11, monté sur le bouchon de l'étui plat.

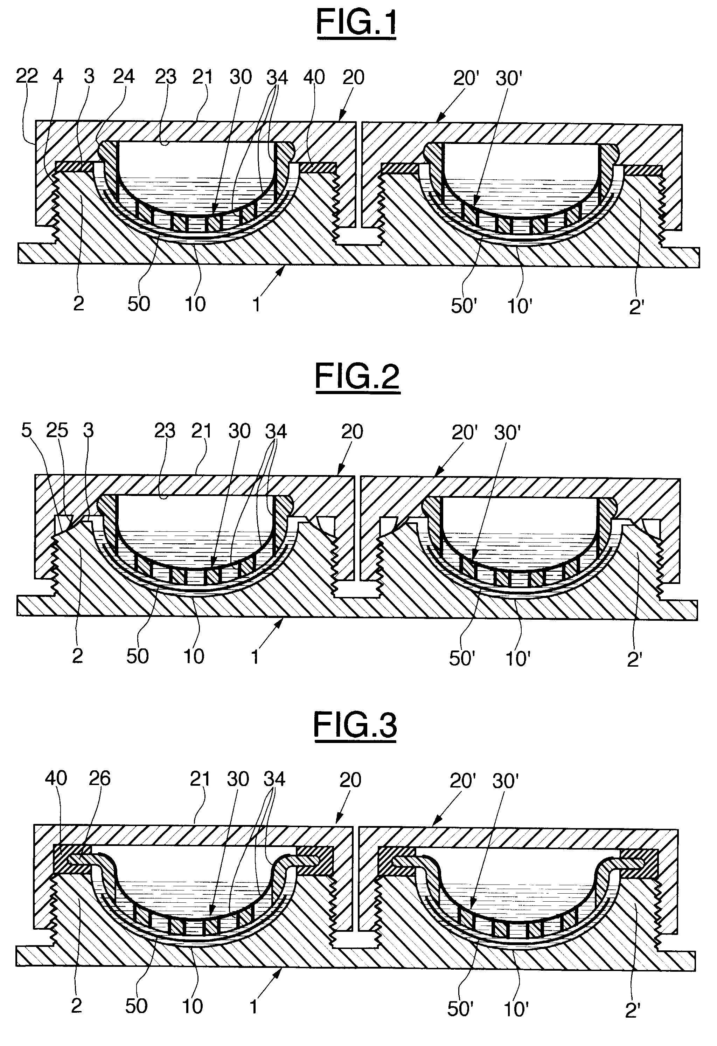

En se référant aux figures où les mêmes numéros de référence correspondent aux mêmes éléments, et plus particulièrement à la figure 1, on a représenté en coupe une réalisation d'un étui plat, selon l'invention.

L'étui plat comprend un corps d'étui 1 comportant deux parois 2, 2' définissant deux chambres de désinfection séparées 10 et 10', deux bouchons 20, 20' et deux éléments de protection 30, 30'.

Les étuis plats, selon l'invention, représentés sur les figures, comportant deux moitiés identiques, la description ci-dessous sera faite en liaison avec une des moitiés de l'étui plat, étant bien entendu que la description d'une moitié s'applique en totalité à l'autre moitié.

La chambre de désinfection 10 est de préférence de forme générale hémisphérique correspondant à la face convexe de la lentille à désinfecter, et est ouverte à son extrémité supérieure. Bien évidemment, la chambre 10 pourrait avoir toute autre forme appropriée telle que cylindrique, polygonale, etc... La paroi 2 définissant la chambre de désinfection 10 se termine, au niveau de l'extrémité ouverte de la chambre de désinfection 10 par une surface d'extrémité annulaire plane 3 et a une surface latérale externe 4 cylindrique pourvue d'un filetage. La chambre de désinfection 10 est fermée par un bouchon amovible 20 ayant une paroi de fond 21 et une paroi latérale cylindrique 22. La surface interne de la paroi latérale 22 du bouchon 20 est pourvue d'un filetage destiné à coopérer avec le filetage sur la surface latérale externe 4 de la paroi 2 de la chambre de désinfection 10 pour maintenir fixement le bouchon sur la chambre. Un évidement cylindrique 23 dont la paroi latérale forme une gorge annulaire 24 est ménagé au centre de la paroi de fond 21 à l'intérieur du bouchon 20.

Un élément de protection 30 est fixé à l'intérieur du bouchon 20 par encliquetage dans l'évidement central 23 de la paroi de fond 21 du bouchon et pend à l'intérieur du bouchon.

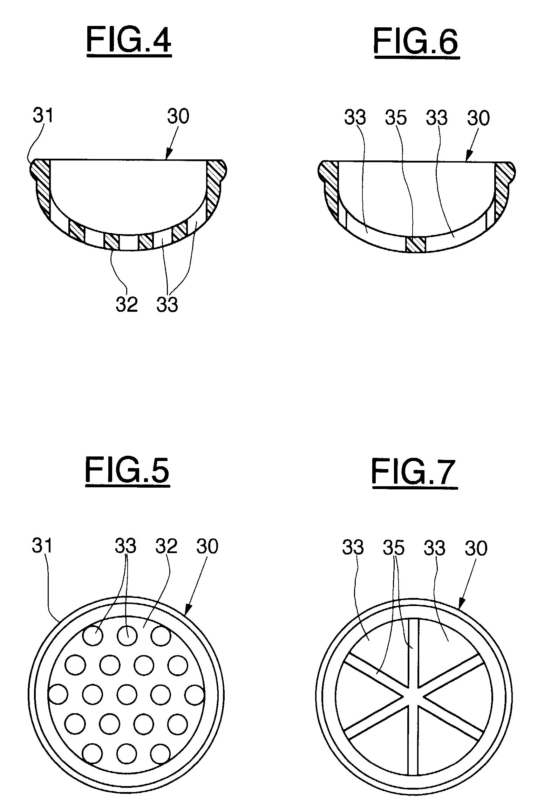

Comme on le voit mieux sur la figure 4, cet élément de protection a la forme générale d'un panier hémisphérique de préférence se conformant à la courbure de la face concave de la lentille à désinfecter et dont l'extrémité supérieure ouverte comporte un rebord annulaire 31 complémentaire de la gorge annulaire 24 de l'évidement 23 dans la paroi de fond 21 du bouchon 20 permettant de fixer par encliquetage l'élément de protection 30 à l'intérieur du bouchon 20. L'élément de protection 30 est pourvu dans sa paroi de fond courbe 32 de passages 33 qui, dans la réalisation représentée comme on le voit sur la figure 5, sont de simples trous.

Un catalyseur 34 est déposé sur la surface interne et, éventuellement, sur les parois latérales des passages 33 de l'élément de protection 30 qui sert ainsi de support pour le catalyseur 34. Ainsi, le catalyseur 34 se trouve protégé de tout contact dommageable, par exemple avec les doigts de l'utilisateur ou avec la lentille lors de l'opération de désinfection.

Bien que l'on ait représenté les passages 33 sous la forme de trous circulaires, ces passages 33 peuvent avoir n'importe quelle forme appropriée, par exemple polygonale, ovale, etc....

De même, le nombre des passages 33 n'est pas critique pour autant qu'il permette une circulation suffisante de la solution de désinfection et un contact effectif avec le catalyseur 34.

Comme le montre la figure 1, un joint annulaire d'étanchéité 40, par exemple un joint en silicone ou en polybutadiène, est disposé entre le bouchon 20 et la surface d'extrémité annulaire plane 3 de la paroi 2 définissant la chambre de désinfection 10.

Le bouchon 20 comporte en outre un moyen de dégazage bien connu dans la technique. Par exemple, ce moyen de dégazage peut être un simple trou ménagé dans la paroi de fond 21 ou encore une ouverture ménagée dans cette paroi de fond 21 et fermée par une membrane perméable aux gaz, en particulier l'oxygène, ou perforée.

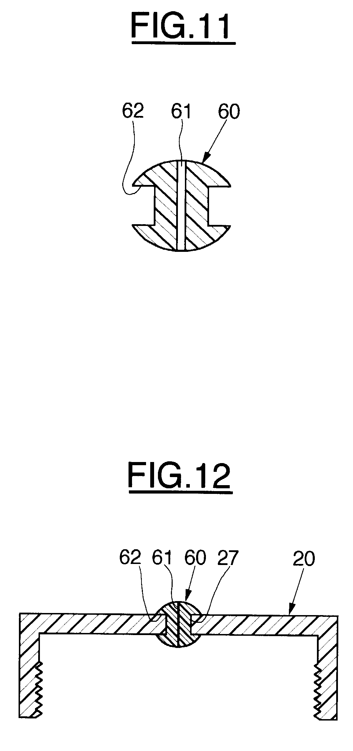

On a représenté aux figures 11 et 12 un moyen de dégazage qui peut être avantageusement utilisé avec l'étui plat selon l'invention. En se référant à la figure 11, qui est une vue en coupe du moyen de dégazage avant son montage sur le bouchon 20 de l'étui, le moyen de dégazage consiste en un manchon 60 constitué d'un matériau souple, par exemple en caoutchouc naturel ou en un élastomère synthétique. Ce manchon souple 60, par exemple de forme générale cylindrique, comporte un passage 61. Une rainure généralement circulaire 62 est également ménagée dans la paroi latérale du manchon 61.

Comme le montre la figure 12, le manchon est disposé dans une ouverture généralement circulaire 27 ménagée dans la paroi de fond 21 du bouchon 20 de l'étui, de telle sorte que le manchon 60 soit maintenu dans le bouchon 20 par engagement des bords de l'ouverture 27 de la paroi de fond 21 du bouchon 20 dans la rainure circulaire 62. En outre, la dimension de l'ouverture 27 dans la paroi de fond 21 du bouchon est telle qu'une fois le manchon 60 disposé dans l'ouverture 27, celui-ci se trouve comprimé et les parois du passage 61 viennent en contact assurant ainsi l'étanchéité aux liquides. Sous l'effet de la pression des gaz engendrés lors de la désinfection, en raison de la nature souple et élastique du matériau constituant le manchon 60, les parois du passage 61 s'écartent laissant s'échapper les gaz.

On va maintenant décrire le fonctionnement de l'étui plat de désinfection selon l'invention, représenté à la figure 1.

Lorsque l'utilisateur souhaite désinfecter des lentilles de contact, il dévisse les couvercles 20 et 20' pour les séparer du corps d'étui 1 en même temps que les éléments de protection 30 et 30' servant également de support de catalyseur. Il introduit alors les lentilles de contact 50, 50' avec leurs faces convexes orientées vers le fond des chambres de désinfection 10, 10'. Il remplit les chambres de désinfection 10, 10' avec la solution de désinfection, par exemple une solution de péroxyde d'hydrogène, puis il visse les bouchons 20, 20', portant les éléments de protection 30, 30' sur les chambres 10, 10'. Comme les éléments de protection 30, 30' pendent à l'intérieur du bouchon, ils sont immergés dans la solution de désinfection. Dès que la solution de désinfection entre en contact avec le catalyseur déposé sur les surfaces internes des éléments de protection 30, 30', la réaction de neutralisation débute. En raison de la forme hémisphérique des éléments de protection 30, 30' correspondant à la face concave des lentilles 50, 50' à désinfecter, les lentilles sont maintenues efficacement dans les chambres de désinfection 10, 10' et le catalyseur 34 se trouve situé au plus près des lentilles et ainsi la réaction de neutralisation s'effectue le plus près possible des surfaces des lentilles 50, 50'. Lors de la réaction de neutralisation, il se dégage de l'oxygène, et les bulles d'oxygène se formant au plus près des lentilles 50, 50' assurent une circulation de la solution à proximité des lentilles et une agitation des lentilles 50, 50', ce qui augmente l'efficacité de la solution de désinfection au niveau des lentilles. En outre, du fait de la présence des passages dans les éléments catalytiques 30, 30', on améliore la circulation de la solution de désinfection et la surface de contact avec le catalyseur et par conséquent l'efficacité de la neutralisation.

En se référant maintenant à la figure 2, on a représenté une autre réalisation d'un étui plat, selon l'invention, qui diffère de l'étui plat de la figure 1 uniquement par les moyens assurant l'étanchéité entre le bouchon 20 et le corps d'étui 1.

Dans cette réalisation, le joint d'étanchéité 40 de l'étui de la figure 1 est remplacé par une lèvre annulaire souple 25 saillant à partir de la surface interne de la paroi de fond 21 du bouchon 20 et entourant l'évidement central 23. Cette lèvre 25 coopère avec la surface d'extrémité annulaire 3 de la paroi 2 définissant la chambre de désinfection 10.

Comme on le voit sur la figure 2, la lèvre annulaire souple 25 a de préférence une section droite ayant la forme générale d'un triangle fixé par sa base à la paroi de fond 21 du bouchon 20.

La surface d'extrémité annulaire 3 de la paroi 2 comprend une surface d'appui annulaire chanfreinée 5 sur laquelle la lèvre 25 du bouchon s'appuie élastiquement lorsque le bouchon 20 est vissé sur l'extrémité ouverte de la chambre de désinfection 10.

De préférence, la paroi latérale interne de la lèvre 25 est légèrement courbe et la surface d'appui annulaire 5 a une courbure complémentaire, ce qui améliore l'étanchéité.

De plus, du fait de sa souplesse, la lèvre 25 permet le dégazage de la chambre de désinfection 10, l'évacuation des gaz au-delà de la lèvre 25 se faisant, soit par l'intermédiaire des filetages, soit par des moyens analogues à ceux décrits en liaison avec la réalisation de la figure 1 et ménagés dans le bouchon 20 à l'extérieur de la lèvre 25.

L'étui plat de la réalisation de cette figure 2 fonctionne comme la réalisation de la figure 1.

On a représenté sur la figure 3 encore une autre réalisation d'un étui plat selon l'invention. Cet étui plat de construction générale analogue à l'étui plat de la réalisation de la figure 1, diffère de cette réalisation en ce que l'élément de protection 30 n'est pas fixé directement au bouchon 20, mais est fixé par un rebord annulaire mince 26 au joint d'étanchéité 40 qui est lui-même fixé à l'intérieur du bouchon 20, par exemple par collage, sur la paroi de fond 21 du bouchon 20. Dans ce cas, il n'est pas nécessaire de prévoir un évidement central dans la paroi de fond 21 du bouchon 20.

L'étui plat de cette réalisation fonctionne comme ceux des réalisations des figures 1 et 2.

Les figures 6 à 9 concernent des variantes de réalisation de l'élément de protection utilisable dans les réalisations d'étui plat des figures 1 et 2.

En se référant aux figures 6 et 7, on a représenté un élément de protection 30 dont la paroi de fond hémisphérique 32 est formée par des croisillons 35, les passages 33 étant formés par les espaces entre les croisillons 35.

Le catalyseur, comme dans l'élément de protection décrit précédemment, est déposé sur les surfaces internes et éventuellement latérales des croisillons 31, l'élément de protection servant également dans ce cas de support de catalyseur.

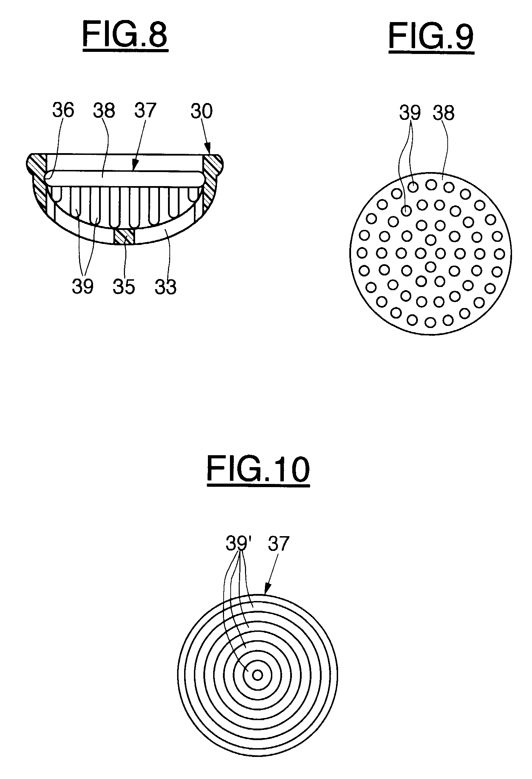

La figure 8 représente une variante de réalisation dans laquelle le support de catalyseur est formé par un élément rapporté 37 fixé à l'intérieur de l'élément de protection 30. L'élément de protection 30, dans le cas représenté, est analogue à l'élément de protection de la figure 6, mais comporte sur sa surface interne à proximité de son extrémité supérieure ouverte une rainure annulaire 36. L'élément rapporté 37 servant de support de catalyseur est constitué d'un disque 38 portant sur une de ses faces des picots 39. Cet élément rapporté 37 est disposé à l'intérieur de l'élément de protection 30, de telle sorte que les picots 39 soient orientés vers la paroi de fond 32 de l'élément de protection 30, et est maintenu dans l'élément de protection 30 par encliquetage de la périphérie du disque 38 dans la rainure annulaire 36 de l'élément de protection 30.

Comme le montre la figure 9, les picots 39 peuvent être répartis en cercles concentriques et ont de préférence une hauteur qui croît depuis la périphérie du disque 38 vers son centre de manière à épouser la forme hémisphérique de l'élément de protection 30 lorsque l'élément rapporté 37 servant de support de catalyseur est disposé dans l'élément de protection 30.

Dans cette réalisation, le catalyseur est déposé sur les surfaces externes des picots 39 pour achever l'élément catalytique.

Avec une telle structure, le catalyseur est totalement protégé de tout contact dommageable par l'élément de protection, tout en étant à proximité étroite des lentilles à désinfecter lors de l'utilisation.

La figure 10 est une vue de dessous d'une autre réalisation d'un élément rapporté 37 dans lequel on a remplacé les picots 39 par des nervures circulaires concentriques 39'. De préférence, également dans cette réalisation, les nervures 39' ont des hauteurs croissantes depuis la périphérie du disque jusqu'à son centre de manière à épouser la forme hémisphérique de l'élément de protection 30.

Dans cette dernière réalisation, le catalyseur est fixé sur les surfaces des nervures 39'.

Bien que sur les figures 4 à 8 on ait représenté des éléments de protection pour la réalisation d'un étui plat selon la figure 1 ou 2, ces éléments de protection peuvent également être adaptés pour la réalisation de la figure 3.

De même, on peut utiliser l'élément de protection 30 de la réalisation de la figure 4 avec un élément rapporté 37 servant de support de catalyseur comme dans la réalisation de la figure 8.

## Revendications

1. Etui plat pour la désinfection de lentilles de contact ayant une face convexe et une face concave, au moyen d'une solution oxydante à base de péroxyde d'hydrogène, comprenant :
- un corps d'étui (1) comportant au moins une paroi (2) définissant une chambre de désinfection (10) ouverte à une extrémité et destinée à recevoir une lentille (50) à désinfecter;
- au moins un bouchon amovible (20) destiné à être fixé sur l'extrémité ouverte de la chambre de désinfection (10);
- au moins un élément catalytique de neutralisation de la solution de désinfection solidaire du bouchon (20) et apte à plonger dans la solution de désinfection lorsque le bouchon est fixé au corps d'étui, et comprenant un support de catalyseur (30, 37) et un catalyseur (34); et
- au moins un élément de protection (30) solidaire du bouchon (20), ayant une paroi de fond (32) pourvue de passages (33) pour la solution de désinfection, faisant barrière entre la lentille (50) et l'élément catalytique tout en assurant un bon maintien de la lentille (50).

2. Etui plat selon la revendication 1, caractérisé en ce que l'élément de protection (30) est de forme générale hémisphérique correspondant à la face concave de la lentille (50).

3. Etui plat selon la revendication 1 ou 2, caractérisé en ce que la chambre de désinfection (10) a une forme générale hémisphérique correspondant à la face convexe de la lentille à désinfecter.

4. Etui plat selon l'une quelconque des revendications 1 à 3, caractérisé en ce que l'élément de protection (30) est fixé au bouchon (20) par encliquetage.

5. Etui plat selon l'une quelconque des revendications 1 à 3, caractérisé en ce que l'élément de protection (30) est fixé à un joint d'étanchéité annulaire (40) lui-même fixé au bouchon (20).

6. Etui plat selon l'une quelconque des revendications 1 à 4, caractérisé en ce que le bouchon (20) comporte une lèvre annulaire souple d'étanchéité (25) destinée à s'appuyer sur une surface d'appui annulaire chanfreinée (5) de la paroi (2) définissant la chambre de désinfection (10).

7. Etui plat selon l'une quelconque des revendications précédentes, caractérisé en ce que les passages (33) dans l'élément de protection (30) sont des trous.

8. Etui plat selon l'une quelconque des revendications 1 à 6, caractérisé en ce que l'élément de protection (30) comprend une paroi de fond (32) hémisphérique formée par des croisillons (35), lesdits passages (33) étant formés par les espaces entre les croisillons.

9. Etui plat selon la revendication 7 ou 8, caractérisé en ce que le support de catalyseur est constitué par les surfaces non exposées de l'élément de protection (30) sur lesquelles est déposé le catalyseur (34).

10. Etui plat selon l'une quelconque des revendications 1 à 6, caractérisé en ce que le support de catalyseur est un élément rapporté (37) fixé à l'intérieur de l'élément de protection (30).

11. Etui plat selon la revendication 10, caractérisé en ce que l'élément rapporté (37) comprend un disque de support (38) dont une face comporte des éléments saillants (39, 39') sur lesquels est fixé le catalyseur, cet élément rapporté (37) étant fixé à l'intérieur de l'élément de protection (30) de sorte que les éléments saillants (39, 39') soient orientés vers la paroi de fond (32) de l'élément de protection (30).

12. Etui plat selon la revendication 11, caractérisé en ce que les éléments saillants (39, 39') ont une hauteur qui croît de la périphérie du disque (38) vers son centre.

13. Etui plat selon la revendication 11 ou 12, caractérisé en ce que les éléments saillants sont des picots (39) ou des nervures circulaires (39').

14. Etui plat selon l'une quelconque des revendications précédentes, caractérisé par le fait qu'il comprend deux parois (2, 2') définissant deux chambres de désinfection (10, 10'), deux bouchons (20, 20'), deux éléments de protection (30, 30') et deux éléments catalytiques.
